# EUROPEAN PATENT APPLICATION

(11) **EP 4 450 646 A2**
(43) Date of publication of application: **23.10.2024**
(21) Application number: 24187116.9
(22) Date of filing: 21.06.2018
(51) Int. Cl.: C12Q 1/6869

(54) **METHOD FOR INVESTIGATING MOLECULES SUCH AS NUCLEIC ACIDS**

(30) Priority: 21.06.2017 EP 17177204; 18.12.2017 EP 17208257
(62) Divisional of application: 21180651.8
(71) Applicant: Lightcast Discovery Ltd, Cambridge CB3 0FA (GB)
(72) Inventor: FRAYLING, Cameron Alexander, Cambridge, CB3 0FA (GB); CUNHA, Pedro, Cambridge, CB3 0FA (GB); ISAAC, Thomas Henry, Cambridge, CB3 0FA (GB)
(74) Representative: Stratagem IPM Limited

(57) **Abstract**

A method for carrying out a chemical transformation of a target molecule immobilised at a given location on a solid support is provided. The method is characterised by the steps of (a) generating a stream of microdroplets each comprised of a medium capable of causing the chemical transformation to occur; (b) contacting each microdroplet in turn with the target molecule at the given location and for a given period of time under conditions where the chemical transformation can occur, wherein the method further comprising the step of synchronising the microdroplets flow over the particle; (c) at the end of the given period removing the microdroplet from the given location; wherein the microdroplets are caused to move over a surface chemically modified to have areas of locally increased level of wettability and wherein such an increase in wettability is used to mediate the wetting of the microdroplet around the target molecule; and wherein the electrowetting or optically-mediated electrowetting effects are used to locally modify the wettability of a region of the surface around the particle.

## Description

This invention relates *inter alia* to a method for investigating a nucleic acid molecule; in particular a method for sequencing DNA or RNA either of natural or synthetic origin.

In our previous applications WO 2014/053853, WO 2014/053854, WO2014/167323, WO2014/167324 and WO2014/111723, we have described a new sequencing method which involves progressive digestion of a polynucleotide analyte to generate an ordered stream of single nucleotides, preferably a stream of single nucleoside triphosphates, each of which can be captured one-by-one into corresponding microdroplets in a microdroplet stream. Thereafter, each microdroplet can be chemically and/or enzymatically manipulated to reveal the particular single nucleotide it originally contained. In one embodiment, these chemical and/or enzymatic manipulations comprise a method involving the use of one or more multi-component oligonucleotide probe types each of which is adapted to be able to selectively capture one of the single nucleotide types from which the analyte is constituted. Typically, in each of such probe types, one of the oligonucleotide components comprises characteristic fluorophores and in the probe's unused state the ability of these fluorophores to fluoresce remains extinguished by virtue of the presence of quenchers located close-by or by self-quenching. In use, when the probe has captured its corresponding single nucleotide, it is rendered susceptible to subsequent exonucleolysis or endonucleolysis thereby liberating the fluorophores from the quenchers and/or each other enabling them to fluoresce freely. By this means, the nature of the original single nucleotide present in each droplet can be inferred indirectly by spectroscopic means.

In designing sequencing devices using this method, it may be necessary to manipulate many thousands of microdroplets ensuring for example that they can be reliably delivered to locations where they can be coalesced with others and/or their contents analysed for the presence or absence of fluorescence.

One possible way of doing this is to establish pathways on a substrate along which the microdroplets can be propelled by electrowetting forces. Devices for manipulating relatively large droplets in this way have been previously described in the art; see for example US6565727, US20130233425 and US20150027889. Typically, this is achieved by causing the droplets, for example in the presence of an immiscible carrier fluid, to travel through a microfluidic space defined by two opposed walls of a cartridge or tubing. Embedded in the walls of the cartridge or tubing are electrodes covered with a dielectric layer each of which is connected to an AC biasing circuit capable of being switched on and off rapidly at intervals to modify the electrowetting characteristics of the layer. This gives rise to localised directional capillary forces that can be used to steer the droplet along a given path.

A variant of this approach, based on optically-mediated electrowetting, has been taught in for example US20030224528, US20150298125 and US20160158748. In particular, the first of these three patent applications discloses various microfluidic devices which include a microfluidic cavity defined by first and second walls and wherein the first wall is of composite design and comprised of substrate, photoconductive and insulating (dielectric) layers. Between the photoconductive and insulating layers is disposed an array of conductive cells which are electrically isolated from one another and coupled to the photoactive layer and whose functions are to generate corresponding discrete droplet-receiving locations on the insulating layer. At these locations, the surface tension properties of the droplets can be modified by means of electrowetting forces. The conductive cells may then switched on and off by light impinging on the photoconductive layer. This approach has the advantage that switching is made much easier and quicker although its utility is to some extent still limited by the arrangement of the electrodes. Furthermore, there is a limitation to the speed at which droplets can be moved and the extent to which the actual droplet pathway can be varied.

A double-walled embodiment of this latter approach has been disclosed in University of California at Berkeley thesis UCB/EECS-2015-119 by Pei. Here, a cell is described which allows the manipulation of relatively large droplets in the size range 100-500µm using optically-mediated electrowetting across a surface of Teflon AF deposited over a dielectric layer. A light-pattern over un-patterned electrically biased amorphous silicon is described in schematic form. However, in the scheme shown the dielectric layer is thin (100nm) and disposed only on the wall bearing the photoactive layer.

Microfluid Nanofluid (2016) 20: 123 teaches electrode-assisted trapping and release of droplets on hydrophilic patches in a hydrophobic microchannel.

In our recently filed European patent application 17180391.9 we have described a nucleic acid investigative device which is capable of manipulating many thousands of microdroplets simultaneously in accordance with the requirements of a fast, reliable single nucleotide detection method. It has the advantage of being easily reconfigurable by the application of computer software making it very versatile; for example by allowing the user to readily adapt it for optimum accuracy, throughput or detecting particular epigenetic modifications. We have now also developed a corresponding investigative method a feature of which is that the initial analyte digestion step is carried out by causing a particle-supported analyte to interact directly with the components of a stream of aqueous microdroplets containing the digesting medium generated upstream (including but not limited to at a separate location; for example upstream of a chip where the digestion takes place) in an immiscible carrier medium. By this means, the digestion step can be performed 'on-particle' and 'in-droplet' thereby increasing significantly the accuracy and reliability of this digestion step.

At its broadest scope, and recognising that this method is widely applicable, there is provided, according to a first aspect of the invention, a method for manipulating a microdroplet of a reaction medium in an immiscible carrier medium with a target molecule bound to a solid support for the purposes of effecting a chemical transformation characterised by the steps of (a) bringing the microdroplet into contact with the solid support under conditions where the microdroplet and solid support are caused to combine, (b) allowing the reaction medium to react with the target molecule and (c) thereafter exerting a force to induce the reaction medium to become detached from the solid support and reform a microdroplet in the carrier fluid.

In one version of this method, there is provided a method for carrying out a chemical transformation of a target molecule immobilised at a given location on a solid support comprising a particle characterised by the steps of (a) generating a stream of microdroplets each comprised of a medium capable of causing the chemical transformation to occur; (b) contacting each microdroplet in turn with the target molecule at the given location and for a given period of time under conditions where the chemical transformation can occur and (c) at the end of the given period removing the microdroplet from the given location.

Suitably, these method further comprises the step of carrying a reformed microdroplet away from the vicinity of the solid support which is depleted in the components of the reaction medium. Preferably, the force exerted is an electrowetting force which is applied a various points or zones on a pathway which includes the location of the solid support. This electrowetting force may be generated electrically or optically.

In one embodiment, the reaction medium employed is for transforming a nucleic acid analyte and the target molecule is a polynucleotide; for example the synthesis of a nucleic acid (e.g. RNA or DNA) using a single-stranded polynucleotide as a primer/template. In such an embodiment, the microdroplets applied to the solid support will comprise a source of nucleotide monomers, at least one polymerase and those other components which are conventional required in the art.

In another embodiment, the method is useful for investigating the molecular structure of a biopolymer such as a nucleic acid (e.g. RNA or DNA) or a protein. For example, according to a second aspect of the present invention, there is provided a method for investigating a target molecule comprising a biopolymer characterised by comprising the steps of:
- causing a stream of microdroplets of the digesting medium in the immiscible carrier medium to contact the biopolymer one by one in a pathway thereby allowing the biopolymer to be progressively digested within the microdroplets into its constituent single monomer units;
- removing the microdroplets from around the biopolymer wherein at least some of the microdroplets contain a single monomer unit;
- further transporting the microdroplets along the pathway to a detection zone and
- identifying the monomer units in the microdroplets in the detection zone and inferring their chemical structure therefrom.

In one embodiment of the first and second aspects, the target molecule/biopolymer is a nucleic acid analyte and the digesting medium is one capable of transforming a polynucleotide. For example, it may contain a polymerase and a cofactor which is preferably a polyphosphate anion or an analogue thereof. Most preferably the polyphosphate is pyrophosphate leading to the generation of nucleotide triphosphates. In another embodiment, the digesting medium comprises an exonuclease which digests the analyte into nucleotide monophosphates. These nucleotide monophosphates may then be converted into their corresponding nucleotide triphosphates by the action of a kinase. Identification of the nucleotide mono- or triphosphates will then comprise identifying the nucleobase associated with each one; for example by the means of nucleobase-selective oligonucleotide capture probes, application of fluorescent labels or directly by detecting characteristic Raman-scattering of the nucleobases enhanced, for example, by surface or plasmonic phenomena. In one embodiment, such an identification step will suitably involve the use of a detection zone employing a source of incident electromagnetic radiation (such as a laser or an LED) and a photodetector for detecting radiation arising from the nucleobases.

In another embodiment, as will be explained in greater detail below, the method of the second aspect of the invention is characterised in that the resulting nucleotides are caused to react in the presence of a polymerase with an oligonucleotide capture system comprised of fluorescence probes which in their unused state are non-fluorescing and which are selective for at least one of the nucleotide types characteristic of the analyte, resulting in the release of fluorophores into a detectable state, and wherein the resulting fluorescence derived from the released fluorophore(s) in each microdroplet is detected using an incident source of first electromagnetic radiation and a photodetector. Suitably the release of such fluorophores is the result of the action of an exonuclease and/or endonuclease and optionally a ligase, such action being catalysed by the capture of the nucleotide being detected. In one embodiment the oligonucleotide capture system is introduced downstream of the analyte digestion site through microdroplet coalescence or through direct injection. In such instance it is preferred that the digesting medium is treated with a pyrophosphatase prior to reaction with the oligonucleotide capture system.

In one preferred version of these embodiments, the biopolymer is a nucleic acid; the monomers are nucleoside triphosphates produced by progressive pyrophosphorolysis and the identification step involves detection of fluorescence generated by exonucleolysis of a used oligonucleotide probe which in its unused state is non-fluorescing and selective for a given nucleobase. Thus, according to a third aspect of the present invention there is provided a method for investigating a nucleic acid analyte characterised by comprising the steps of:
- causing a stream of microdroplets of an aqueous pyrophosphorolysing medium in an immiscible carrier medium to contact the analyte in a pathway; thereby allowing the analyte to be progressively into its constituent single nucleoside triphosphates;
- removing the microdroplets in the carrier medium from around the analyte wherein at least some of the microdroplets contain one of the single nucleoside triphosphates;
- further transporting the microdroplets along the pathway;
- at one or more locations along the pathway or upstream of the pyrophosphorolysis introducing into each microdroplet detection components comprised of (a) an oligonucleotide capture system comprised of fluorescence probes which in their unused state are non-fluorescing, (b) a polymerase, (c) optionally a ligase and (d) an exonuclease and/or an endonuclease capable of causing the fluorophore(s) to be released from the used capture system in a fluorescing state;
- detecting fluorescence derived from the released fluorophore(s) in each microdroplet at a point downstream of the location(s) and the pyrophosphorolysis using an incident source of first electromagnetic radiation and a photodetector.

The methods of these second or third aspects of the present invention are especially suitable for sequencing a double-stranded polynucleotide analyte having a nucleotide chain length which can in principle be unlimited; for example up to and including the many millions of nucleotide base pairs found in a fragment of a genome. In one embodiment, the analyte will therefore be at least 50, preferably at least 150 nucleotide base pairs long; suitably it will be greater than 500, greater than 1000 and in some cases 5000+ nucleotide base pairs long. In one embodiment, the analyte is DNA of natural origin (e.g. genetic material derived from a plant, animal, bacterium or a virus) although the method is equally applicable to the sequencing of partially or wholly synthetic DNA or indeed other nucleic acids made up wholly or in part of nucleotides comprised of nucleobases that are not commonly encountered in nature; i.e. nucleotides having nucleobases other than adenine, thymine, guanine, cytosine and uracil. Examples of such nucleobases include 4-acetylcytidine, 5-(carboxyhydroxylmethyl)uridine, 2-O-methylcytidine, 5-carboxymethylaminomethyl-2-thiouridine, 5-carboxymethylamino-methyluridine, dihydrouridine, 2-O-methylpseudouridine, 2-O-methylguanosine, inosine, N6-isopentyladenosine, 1-methyladenosine, 1-methylpseudouridine, 1-methylguanosine, 1-methylinosine, 2,2-dimethylguanosine, 2-methyladenosine, 2-methylguanosine, 3-methylcytidine, 5-methylcytidine, N6-methyladenosine, 7-methylguanosine, 5-methylaminomethyluridine, 5-methoxyaminomethyl-2-thiouridine, 5-methoxyuridine, 5-methoxycarbonylmethyl-2-thiouridine, 5-methoxycarbonylmethyluridine, 2-methylthio-N6-isopentenyladenosine, uridine-5-oxyacetic acid-methylester, uridine-5-oxyacetic acid, wybutoxosine, wybutosine, pseudouridine, queuosine, 2-thiocytidine, 5-methyl-2-thiouridine, 2-thiouridine, 4-thiouridine, 5-methyluridine, 2-O-methyl-5-methyluridine and 2-O-methyluridine. Where the analyte is RNA similar considerations will apply.

Turning to the preferred embodiment of the third aspect of the invention, in the first step and in a pyrophosphorolysis zone within the pathway, the analyte is progressively pyrophosphorolysed in the 3'-5' direction to generate a stream of single nucleoside triphosphates the order of which corresponds to that of the sequence of the analyte. The pyrophosphorolysis itself is carried out at a temperature in the range 20 to 90°C in the presence of a stream of microdroplets containing an aqueous pyrophosphorolysing medium including an enzyme such as a polymerase. In one embodiment, this medium is buffered and may also contain those other components needed to sustain the pyrophosphorolysis reaction (polymerase, pyrophosphate anion, magnesium cation etc.). In another embodiment, the medium additionally contains one or more of the nucleotide detection components which are further specified below. In yet another embodiment some or all of these components can be introduced at another of various points in the practice of the method; for example, by direct injection using a microdroplet injector or by coalescence of the microdroplets with secondary microdroplets containing the relevant materials. Suitably the microdroplets employed are suspended in an immiscible carrier medium such as a mineral or silicone oil.

Preferably, the method of the invention is operated so that the rate of pyrophosphorolysis is as fast as possible and in one embodiment this rate lies in the range from 1 to 50 single nucleoside triphosphates per second. Further information about the pyrophosphorolysis reaction as applied to the progressive degradation of polynucleotides can be found for example in J. Biol. Chem. 244 (1969) pp. 3019-3028.

In one embodiment, the analyte digestion is carried out by contacting each microdroplet in the stream in turn with a particle to which the analyte has previously been attached. Suitably, such particles will have previously been modified to include one or more analyte-reactive sites to which the analyte is bound by chemical or physical means. In one embodiment, the particle comprises a bead; for example a microbead, made of an inert material such as glass, silica, alumina, a metal or a non-degradable polymer. In another embodiment, the particle has a core of paramagnetic material enabling it to be manipulated magnetically. In another embodiment, the particle may be contained in an initial microdroplet and manipulated to its digestion location by means of conventional or optically-mediated electrowetting. In yet another embodiment, the particle may be held at a given location where digestion is to take place by one or more means including magnetism, electrowetting, suction, physical constriction, or by secondary attachment groups mounted thereon which can chemically or physically bind temporarily to the walls of the pathway; as for example where the pathway is a microfluidic channel or tubing.

There are many ways in which these secondary attachment groups and the analyte-reactive sites can be created. For example, in the case of the former they can be prepared by first partially coating the particles with metal using a known method such as metal vapour deposition, plasma deposition or the like. Thereafter, the metal surface can be chemically modified to introduce one or more first moieties which can reversibly bind to complementary second moieties disposed at a location in the pathway. In one embodiment, these pairs of first and second moieties are chosen so that the bond created between them can be reversibly made and broken at ambient or near ambient temperatures. By this means the particle can be attached at the digestion location, the analyte digested, and thereafter the bead easily detached from the location enabling the latter to then receive another fresh bead. In one embodiment, these pairs of first and second moieties can lead to the formation of protein complexes stable under the conditions of digestion e.g. by using avidin or streptavidin moieties with complementary biotin moieties. In another, these pairs can create a labile chemical bond; as for example in a polyhistidine/chelated metal ion pair (bond broken at low pH or in the presence of imidazole or strong metal chelator); a boronic acid/suitable carbohydrate pair (bond broken at low pH) or a maleimido/selenol pair (bond broken using meta-chloroperoxybenzoic acid). In these cases, the particle can be subsequently detached by modifying the pH or composition of the microdroplets passing over it.

In one preferred embodiment, the pair of moieties mentioned above comprises a polyhistidine moiety comprised of a plurality of histidine residues (preferably greater than six) and a chelator moiety selected from for example a nitrotriacetate (NTA) or iminodiacetate (IDA) salt derivative of a transition metal such as cobalt, copper or nickel. It will be appreciated that these first and second moieties can be deployed on the particle and pathway location respectively either way around.

Likewise, there are many ways in which the analyte-reactive sites can be created. Thus, in another embodiment, an uncoated surface of the particle may be primed with a functionalising agent, for example in the case of a silica, alumina or glass bead, an epoxysilane, an aminohydrocarbylsilane or a mercaptosilane, to create chemically reactive sites to which the analyte can be attached. Thereafter these reactive sites can be treated with a derivative of the analyte which has been correspondingly modified to include a terminal primer-reactive group; for example in one embodiment an amine, succinyl or thiol group. In another embodiment, chemical attachment can take place via ligation to adaptor oligonucleotides or via the types of protein complexes described above.

In yet another embodiment, the particle so primed will comprise a region having only one analyte-reactive site so that only one molecule of the analyte can be attached. This can be important if the analyte is a small polynucleotide fragment since otherwise multiple molecules tend to become attached during preparation which is undesirable. It is of a lesser concern if the polynucleotide fragment is large since steric effects will work to militate against such an outcome. Suitably, the particle will have a maximum diameter in the range 0.5 to 5 microns (µm).

In one preferred embodiment, the particles are spherical beads having a magnetic core; for example those sold under the name Dynabeads^{®}.

It is advantageous in some embodiments of the method where the target analyte is immobilised in such a way that is difficult to wet or too small to wet (such as an analyte which is immobilised on a hydrophobic bead or on a very small bead) to modify the wettability of the surface walls near to the analyte. Such a modification to the wettability can be achieved using a chemical surface coating, such as a coating with PEG-silane. In another embodiment, a temporary modification to the wettability can be induced using one of the electrowetting mechanisms mentioned earlier. By modifying the surface wettability it is possible to improve the reliability of the method as the user can as precisely define the surface area which receives this wettability treatment. Thus, it becomes easier to control the precise volume of fluid which is de-wetted from the surface by onward droplet transport, and the volume of the remaining fluid which might be left behind wetted to the surface. This has the advantage of giving highly reproducible volume exchange between the target analyte and the droplets which would be difficult to achieve with a target of poorly defined surface area such as an isolated bead with a rough surface.

It is a feature of the first step of the method that the digestion of the analyte takes place in a dropletised aqueous medium in which microdroplets suspended in an immiscible carrier medium temporarily wet and de-wet the target bead as they are driven over its surface. By this means, when a nucleotide triphosphate molecule is released from the analyte strand it will become encapsulated and then removed downstream when the microdroplet de-wets or partially de-wets from the particle. In one embodiment the digestion takes place when the particle is in temporary contact with a microdroplet. In yet another, the rate of wetting/dewetting is tuned so that on average more microdroplets pass over the particles than nucleotides are released. In another embodiment the volume of the particle is less that the volume of the microdroplets; for example less than 75% or preferably less than 50% of the volume. In yet another, the particle may retain an aqueous shell as the microdroplets pass over it. In this case it is preferable that the ratio of shell volume to microdroplet volume be as small as possible. For example, if 50% of the microdroplet volume were to remain on the particle, this gives a 50% chance of the nucleotide being carried away with the microdroplet, and 50% chance it remains behind.

It will be appreciated that this microdroplet wetting/de-wetting methodology will have application beyond sequencing if the progressive chemical transformation carried out on the analyte is other than digestion; for example the synthesis of DNA or the synthesis of nanoparticles. The possibilities for such transformations may in one embodiment be further increased by having multiple microdroplet types interact in a stepwise fashion with a particle bearing for example a catalyst, primer or other reactant. Thus, according to another aspect of the present invention there is provided a general method for carrying out a chemical transformation of a target molecule immobilised at a given location characterised by the steps of (a) generating a stream of microdroplets each comprised of a medium capable of causing the chemical transformation to occur; (b) contacting each microdroplet in turn with the target molecule at the given location and for a given period of time under conditions where the chemical transformation can occur and (c) at the end of the given period removing the microdroplet from the given location. Suitably, the microdroplets are suspended in an immiscible carrier of the type described herein and the given location is a particle such as those described above.

To avoid the risk that a given microdroplet contains more than one single nucleotide molecule, it is preferred to synchronise the microdroplet flows over the particle and digestion so that each filled microdroplet generated is separated on average by from 1 to 20 preferably 2 to 10 empty ones. Suitably the microdroplets have a finite volume of less than 500pL (picolitres), preferably less than 65pL, more preferably less than 4pL and even more preferably less than 2pL. Most preferably of all, their volumes are in the range 4fL (femtolitres) to 4pL. In one embodiment, the microdroplet flow rate over the particle is in the range 1 to 1000 microdroplets per second preferably 50 to 500.

After the microdroplets have been removed from the particle and around the analyte, they create an ordered stream some of which now contain the nucleotide constituents of the analyte. This stream of microdroplets is then further transported along the pathway which for example may comprise a microfluidic space such as a microfluidic channel or tubing. Suitably this transportation may use pressure driven flow, or may use electrowetting force; for example by means of a conventional 'electrowetting on dielectric' (eWOD) configuration or preferably one where the electrowetting forces are generated by optical effects. In one preferred embodiment, the pathway is an optically mediated electrowetting microfluidic space constructed of or defined by first and second composite walls comprised of first and second substrates, first and second conductor layers, a photoactive layer and first and second dielectric layers. In another embodiment, this zone comprises a chip or flat cartridge which is hollow and accommodates the microfluidic space. In yet another, at least the first substrate and first conductor layers are transparent enabling light from a source of second electromagnetic radiation (for example multiple laser beams or LED diodes) to impinge directly onto the photoactive layer. In another embodiment, the second substrate, second conductor layer and second dielectric layer are transparent so that the same objective can be obtained. In yet another embodiment all these layers are transparent enabling illumination to occur from either side.

Suitably the first and second substrates are made of a material which is mechanically strong for example glass, metal, semiconductor or an engineering plastic. In one embodiment, the substrates may have a degree of flexibility. In yet another embodiment, the first and second substrates have a thickness in the range 100-1000µm.

The first and second conductor layers are located on one surface of the first and second substrates and are typically very thin; each having a thickness in the range 70 to 250nm, preferably 70 to 150nm. In one embodiment, at least one of these layers is made of a transparent conductive material such as Indium Tin Oxide (ITO), a very thin film of conductive metal such as silver or a conducting polymer such as PEDOT or the like. The layers may be formed as a continuous sheet or a series of discrete structures such as wires. Alternatively, the conductor layer may be a mesh of conductive material with the electromagnetic radiation being directed between the interstices of the mesh.

The photoactive layer is suitably comprised of a semiconductor material which can generate localised areas of charge in response to stimulation by the source of second electromagnetic radiation. Examples include amorphous silicon in a thickness range from 300 to 1000nm. In one embodiment the photoactive layer is activated by the application of visible light.

The photoactive layers in the case of the first wall and optionally the conducting layer in the case of the second wall are coated with dielectric layers which are very thin and typically in the range 120 to 160nm. The dielectric properties of this layer preferably include a high dielectric strength of >10^7 V/m and a dielectric constant of >3. In one embodiment, the dielectric layer is selected from high purity alumina or silica, hafnia or thin non-conducting polymer film.

In another version of this configuration at least the first dielectric layer, preferably both layers, are coated with an anti-fouling layer to assist in establishing the desired microdroplet/carrier fluid/surface contact angle at the various electrowetting locations and additionally to prevent the contents of the microdroplets adhering to the surface and being diminished as the microdroplet is moved along the pathway. If the second wall does not comprise a second dielectric layer then the second antifouling layer may be applied directly onto to the second conductor layer. For optimum performance the antifouling layer should assist in establishing a contact angle in the range 50-70° when measured as an air-liquid-surface three-point interface at 25°C. Dependent on the choice of carrier medium the same contact angle of microdroplets in a similar pathway filled with an aqueous emulsion will be higher; greater than 100°. In one embodiment, these layer(s) have a thickness of less than 50nm and are typically a monomolecular layer. In another, these layers are comprised of a polymer of an acrylate ester such as methyl methacrylate or a derivative thereof substituted with hydrophilic groups; e.g. alkoxysilyl. Preferably either or both of the antifouling layers are hydrophobic to ensure optimum performance.

The first and second dielectric layers and therefore the first and second walls suitably define a pathway comprised of a microfluidic space which is less than 50 preferably less than 10µm in depth and in which the microdroplets are contained. In one embodiment, before they are contained in the microdroplet space, the microdroplets have an intrinsic diameter which is more than 10% greater suitably more than 20% greater, than one dimension of the microdroplet space. By this means, within the pathway the microdroplets are caused to experience compression leading to enhanced electrowetting performance for certain operations through the addition of surface potential energy above that of the uncompressed natural state of the microdroplets.

In another embodiment, the pathway may include one or more spacers for holding the first and second walls apart to a predetermined separation. Options for spacers include beads or pillars, ridges or the like created from an intermediate layer, for example a resist layer which has been produced by photo-patterning. Various spacer geometries can also be used to define spaces such as narrow channels, tapered channels or partially enclosed channels which are defined by lines of pillars. By careful design, it is possible to use these structures to aid in the deformation of the microdroplets, to subsequently perform microdroplet splitting and to carry out operations on the deformed microdroplets.

The distance between the first and second walls affects the level of deformation of the microdroplets; adding or removing surface drag which impedes microdroplet motion. By structuring the device such that the spacing between the first and second walls varies across the device it is possible to have a pathway of at least one variable dimension in which the profile is tailored for different microdroplet operations.

The first and second walls are biased using a source of AC power attached to the conductor layers to provide a voltage potential difference therebetween; suitably in the range 10 to 50 volts AC.

The sequencing device of the present invention further includes a source of second electromagnetic radiation having a wavelength in the range 400-1000nm and an energy higher than the bandgap of the photoexcitable layer. Suitably, the photoactive layer will be activated at electrowetting locations where the incident intensity of the radiation employed is in the range 0.01 to 0.2 Wcm⁻². The source of electromagnetic radiation is, in one embodiment, highly attenuated and in another pixelated so as to produce corresponding photoexcited regions on the photoactive layer which are also pixelated. By this means corresponding pixelated electrowetting locations on the first dielectric layer are induced. In contrast to the design taught in US20030224528, these points of pixelated electrowetting are not associated with any corresponding permanent structure in the first wall as the conductive cells are absent. As a consequence, in the device of the present invention and absent any illumination, all points on the surface of first dielectric layer have an equal propensity to become electrowetting locations. This makes the device very flexible and the electrowetting pathways highly programmable. To distinguish this characteristic from the types of permanent structure taught in the prior art we have chosen to characterise the electrowetting locations generated in our device as 'ephemeral' and the claims of our application should be construed accordingly.

The optimised pathway design taught here is particularly advantageous in that the resulting composite stack has the anti-fouling and contact-angle modifying properties from the coated monolayer (or very thin functionalised layer) combined with the performance of a thicker intermediate layer having high-dielectric strength and high-dielectric constant (such as aluminium oxide or hafnia). The resulting layered structure is highly suitable for the manipulation of very small volume microdroplets, such as those having diameter less than 10µm, for example in the range 2 to 8, 2 to 6, 2 to 5 or 2 to 4µm. In one preferred embodiment, these ranges give rise to microdroplets having a volume in the range from 10fL to 1pL For these extremely small microdroplets, the performance advantage of having minimal thickness of total non-conducting stack above the photoactive layer is extremely advantageous, as the droplet dimensions start to approach the thickness of the dielectric stack and hence the field gradient across the droplet (a requirement for electrowetting-induced motion) is reduced for the thicker dielectric.

Where the source of second electromagnetic radiation is pixelated it is suitably supplied either directly or indirectly using a reflective screen or digital micromirror device (DMD) illuminated by light from for example LEDs. This enables highly complex patterns of ephemeral electrowetting locations to be rapidly created and destroyed in the first dielectric layer thereby enabling the microdroplets to be precisely steered along the electrowetting pathways using closely-controlled electrowetting forces. This is especially advantageous when the aim is to manipulate many thousands of such microdroplets simultaneously along multiple electrowetting pathways. Such electrowetting pathways can be viewed as being constructed from a continuum of virtual electrowetting locations on the first dielectric layer.

The points of impingement of the sources of second electromagnetic radiation on the photoactive layer can be any convenient shape including the conventional circular. In one embodiment, the morphologies of these points are determined by the morphologies of the corresponding pixelations and in another correspond wholly or partially to the morphologies of the microdroplets in the pathway. In one preferred embodiment, the points of impingement and hence the electrowetting locations may be crescent-shaped and orientated in the intended direction of travel of the microdroplet in the pathway. In another embodiment, the second wall also includes a photoactive layer which enables further ephemeral electrowetting locations to be induced on the second dielectric layer; either by means of the same source of electromagnetic radiation or a second source of the type described herein. Suitably, the electrowetting locations themselves are smaller than the microdroplet surface adhering to the first wall and give a maximal field intensity gradient across the contact line formed between the droplet and the surface dielectric. The addition of a second photoactive layer enables the possibility of transitioning the wetting edge from the upper to the lower surface of the pathway, and the targeted application of alternative or additional electrowetting forces to each microdroplet.

In one embodiment, the means for manipulating the points of impingement of the second electromagnetic radiation on the photoactive layer is adapted or programmed to produce a plurality of concomitantly-running, for example parallel, first electrowetting pathways on the first and optionally the second dielectric layers. In another embodiment, it is adapted or programmed to also produce a plurality of third electrowetting pathways on the first and optionally the second dielectric layers which intercept with the first electrowetting pathways to create at least one microdroplet-coalescing location where different primary and secondary microdroplets travelling along the different pathways can be caused to coalesce. The first and third electrowetting pathway may intersect at right-angles to each other or at any angle thereto including head-on. This embodiment is especially useful where it is desired to deliver the detection components (see below) into the microdroplets in the pathway by droplet coalescence. In yet another embodiment, where multiple first, second and third electrowetting pathways are employed, the means for manipulating the points of impingement is suitably controlled by a microprocessor which not only creates the pathways along which the microdroplets pass but also synchronises the movement of each microdroplet relative to each other one.

As mentioned above, in some embodiments the method of the invention also includes the step of introducing at one or more locations along the pathway nucleotide detection components comprised of (a) an oligonucleotide capture system comprised of the fluorescent probe types specified below and/or in our earlier patent applications (or probes having an equivalent function); (b) a polymerase, (c) optionally a ligase and (d) an exonuclease and/or endonuclease capable of causing the fluorophore(s) to be released from the used capture system in a fluorescing state. In one embodiment, some or all of these components are introduced together or in stages into the microdroplets at location(s) along the pathway or upstream thereof (see above); for example by being present in the original microdroplet medium. Introduction of these detection components can be achieved at one or more locations by for example direct injection using a microdroplet injector or by coalescence of the microdroplets with secondary microdroplets containing the relevant materials. In one preferred embodiment, a pyrophosphatase is introduced into the microdroplets downstream of the analyte and upstream of the first point of introduction of the detection components.

Various capture systems may be employed in accordance with our earlier patents. For example, in one embodiment they are selected from systems comprising (a) a first single-stranded oligonucleotide labelled with characteristic fluorophores in an undetectable state and (b) second and third single-stranded oligonucleotides capable of hybridising to complementary regions on the first oligonucleotide.

In another embodiment, they are selected from systems comprising (a) a first single-stranded oligonucleotide including a restriction endonuclease nicking-site, a single nucleotide capture site for capturing the single nucleoside triphosphate and oligonucleotide regions juxtaposed either side of the nicking-site bearing respectively at least one fluorophore and at least one quencher so as to render the fluorophores quenched and (b) second and third single-stranded oligonucleotides capable of hybridising to complementary regions on the first oligonucleotide either side of the capture site.

In another, they are selected from systems comprising either (a) a first single-stranded oligonucleotide including an exonuclease blocking-site, a restriction endonuclease recognition-site located on the 5' side of the blocking-site and including a single nucleotide capture-site for capturing the single nucleoside triphosphate, and at least one fluorophore region located on the 5' side of the recognition-site arranged so as to render the fluorophore(s) quenched and (b) a second and optionally a third single-stranded oligonucleotide each separate from the first oligonucleotide and capable of hybridising to complementary regions on the first oligonucleotide flanking the 3' and 5' sides of the capture-site or (a) a first single-stranded oligonucleotide including an exonuclease blocking-site, a restriction endonuclease recognition-site located on the 3' side of the blocking-site and including a single nucleotide capture-site for capturing the single nucleoside triphosphate, and at least one fluorophore region located on the 3' side of the recognition-site arranged so as to render the fluorophore(s) quenched and (b) a second and optionally a third single-stranded oligonucleotide each separate from the first oligonucleotide and capable of hybridising to complementary regions on the first oligonucleotide flanking the 3' and 5' sides of the capture-site.

In another, they are selected from systems comprising either (i) two components comprising; (a) a first oligonucleotide comprising a double-stranded region and a single-stranded region and (b) a second single-stranded oligonucleotide whose nucleobase sequence is at least partially complimentary to that of the single-stranded region of the first oligonucleotide or (ii) a single oligonucleotide comprising a single-stranded nucleotide region the ends of which are attached to two different double-stranded oligonucleotide regions; and wherein the oligonucleotide capture systems are labelled with fluorophores in an undetectable state

In yet another, they are selected from systems comprising a single-stranded nucleotide region the ends of which are attached to double-stranded oligonucleotide regions wherein at least one of the oligonucleotide regions comprises fluorophores in an undetectable state.

After introduction of the detection components and preferably after a period of incubation during which a fluorescence signal develops, the microdroplets are interrogated downstream using a detection system comprising an incident source of first electromagnetic radiation and a photodetector. It will be appreciated that in some embodiments the first and second sources of electromagnetic radiation may be the same. By this means, fluorescing fluorophores released as the used oligonucleotide capture system undergoes exonucleolysis and or endonucleolysis are detected and the nature of the nucleoside triphosphate contained with each microdroplet determined. In one embodiment, this detection will occur in a detection zone comprising a surface provided with detection locations at the end of the pathway to which the microdroplets are ultimately driven by one or a combination of pressure driven flow, conventional electrowetting, or optically-mediated EWOD. Such a surface may be profiled with structures such as wells and the like to create these final destination locations. In another embodiment, the detection step involves introducing the microdroplets into a detection zone comprising an array of capillary tubes through which each microdroplet is stacked and travels before being interrogated by the fluorescence-detection system at detection locations located at the point of exit.

In one embodiment, the detection system is comprised of one or more sources of first electromagnetic radiation, for example one of more lasers or LEDs, to interrogate the contents of each microdroplet and one or more photodetectors or equivalent devices tuned to the characteristic fluorescence wavelength(s) or wavelength envelope(s) of the various fluorophores employed in the particular capture system being used. Any emitted fluorescence detected by the photodetector(s) thereafter gives rise to an electrical signal which can be processed and analysed in a computer using known algorithms to reveal data characteristic of the sequence of the analyte.

In one embodiment of the preferred third aspect of the invention there is provided a method for pyrophosphorolysing a nucleic acid analyte characterised by the step of contacting in turn each microdroplet in a stream of aqueous microdroplets in an immiscible carrier medium and containing a pyrophosphorolysing enzyme with a particle to which the analyte is attached whereby the enzyme is caused to release a nucleoside triphosphate molecule from the analyte into at least some of the microdroplets before they are removed from around the particle.

In another, this method is characterised in that the oligonucleotide capture system comprises (a) a first single-stranded oligonucleotide labelled with characteristic fluorophores in an undetectable state and (b) second and third single-stranded oligonucleotides capable of hybridising to complementary regions on the first oligonucleotide.

In another, this method is characterised in that the oligonucleotide capture system comprises (a) a first single-stranded oligonucleotide including a restriction endonuclease nicking-site, a single nucleotide capture site for capturing the single nucleoside triphosphate and oligonucleotide regions juxtaposed either side of the nicking-site bearing respectively at least one fluorophore and at least one quencher so as to render the fluorophores quenched and (b) second and third single-stranded oligonucleotides capable of hybridising to complementary regions on the first oligonucleotide either side of the capture site.

In another, this method is characterised in that the oligonucleotide capture system comprises either (a) a first single-stranded oligonucleotide including an exonuclease blocking-site, a restriction endonuclease recognition-site located on the 5' side of the blocking-site and including a single nucleotide capture-site for capturing the single nucleoside triphosphate, and at least one fluorophore region located on the 5' side of the recognition-site arranged so as to render the fluorophore(s) quenched and (b) a second and optionally a third single-stranded oligonucleotide each separate from the first oligonucleotide and capable of hybridising to complementary regions on the first oligonucleotide flanking the 3' and 5' sides of the capture-site or (a) a first single-stranded oligonucleotide including an exonuclease blocking-site, a restriction endonuclease recognition-site located on the 3' side of the blocking-site and including a single nucleotide capture-site for capturing the single nucleoside triphosphate, and at least one fluorophore region located on the 3' side of the recognition-site arranged so as to render the fluorophore(s) quenched and (b) a second and optionally a third single-stranded oligonucleotide each separate from the first oligonucleotide and capable of hybridising to complementary regions on the first oligonucleotide flanking the 3' and 5' sides of the capture-site.

In yet another, this method is characterised in that the oligonucleotide capture system comprises either (i) two components comprising; (a) a first oligonucleotide comprising a double-stranded region and a single-stranded region and (b) a second single-stranded oligonucleotide whose nucleobase sequence is at least partially complimentary to that of the single-stranded region of the first oligonucleotide or (ii) a single oligonucleotide comprising a single-stranded nucleotide region the ends of which are attached to two different double-stranded oligonucleotide regions; and wherein the oligonucleotide capture systems are labelled with fluorophores in an undetectable state.

In yet another, this method is characterised in that the oligonucleotide capture system comprises a single-stranded nucleotide region the ends of which are attached to double-stranded oligonucleotide regions wherein at least one of the oligonucleotide regions comprises fluorophores in an undetectable state.

A method of the present invention suitable for sequencing a DNA analyte according to the preferred third aspect of the invention is now illustrated by the following Figures and associated description.

Figure 1 shows a plan of a microfluidic chip comprised of a microdroplet preparation zone 1 and a microdroplet manipulation zone 3 integrated into a single chip made of transparent plastic. 1 comprises regions containing fluid 7, 8 and 9 attached to inlets 4, 5 and 6 which respectively introduce into the chip a pyrophosphorolysing stream, an inorganic pyrophosphatase stream and a stream of the various detection chemicals and enzymes required to identify nucleoside triphosphates in accordance with one of our earlier patent applications. These streams are then delivered respectively to orifices 7a, 8a and 9a from which various microdroplets 7b, 8b and 9b are produced (for example using a droplet-dispensing head or by cutting from a larger intermediate droplet). 10 is a reservoir containing paramagnetic-polymer composite microbeads 11 to some or all of which are attached a single molecule of the polynucleotide analyte to be sequenced. Each of 11 is transported in microdroplets along electrowetting pathway 12 to a location 7c where it is held by magnetism and contacted with a stream of 7b. At this location, the analyte attached to 11 is progressively pyrophosphorolysed at a rate such that after each 7b is disengaged from 11 it is either empty or contains only one single nucleoside triphosphate molecule. After disengagement, 7b along with microdroplets 8b and 9b, are caused to move to 3 where they are manipulated along various optically-mediated electrowetting paths (defined here by square electrowetting locations 25) at a temperature of 30-40°C in accordance with the scheme illustrated in Figure 2. In the process, 7b and 8b are first caused to coalesce at first coalescing points 12 to generate intermediate microdroplets 13 which are thereafter caused to coalesce with 9b at second coalescing points 14 to generate a plurality of streams of final microdroplets 15 which move forward through an incubation region 16 maintained at a temperature in the range 60-75°C in order to allow the contents to incubate and the necessary chemical and enzymatic reactions to take place. Thereafter, 15 are transported to final locations in detection zone 2 where they are interrogated with light from a LED source and any fluorescence emitted by each microdroplet detected using a photodetector. The output of the photodetector is a data stream corresponding to the sequence of the analyte which can be analysed using known sequencing algorithms.

Figure 2 shows a partial, sectional view of 3 illustrating how the various microdroplets are manipulated. 3 comprises top and bottom glass or transparent plastic plates (17a and 17b) each 500µm thick and coated with transparent layers of conductive Indium Tin Oxide (ITO) 18a and 18b having a thickness of 130nm. Each of 18a and 18b is connected to an AC source 19 with the ITO layer on 18b being the ground. 18b is coated with a layer of amorphous silicon 20 which is 800nm thick. 18a and 20 are each coated with a 160nm thick layer of high purity alumina or hafnia 21a and 21b which are in turn coated with a monolayer of poly(3-(trimethoxysilyl)propyl methacrylate) 22 to render their surfaces hydrophobic. 21a and 21b are spaced 3µm apart using spacers (not shown) so that the microdroplets undergo a degree of compression when introduced into this space. An image of a reflective pixelated screen, illuminated by an LED light source 23 is disposed generally beneath 17b and visible light (wavelength 660 or 830nm) at a level of 0.01Wcm² is emitted from each diode 24 and caused to impinge on 20 by propagation in the direction of the multiple upward arrows through 17b and 18b. At the various points of impingement, photoexcited regions of charge 26 are created in 20 which induce modified liquid-solid contact angles in 21b at corresponding electrowetting locations 25. These modified properties provide the capillary force necessary to propel the microdroplets from one point 25 to another. 23 is controlled by a microprocessor (not shown) which determines which of 26 in the array are illuminated at any given time using a pre-programmed algorithm. By this means, the various microdroplets can be moved along the various pathways shown in Figure 1 in a synchronised way.

Figure 3 shows a top-down plan of a typical microdroplet (here 7b) located at a location 25 on 21b with the dotted outline 7' delimiting the extent of touching. In this example, 26 is crescent-shaped in the direction of travel of 7b.

## Claims

1. A method for carrying out a chemical transformation of a target molecule immobilised at a given location on a solid support comprising a particle **characterised by** the steps of
(a) generating a stream of microdroplets each comprised of a medium capable of causing the chemical transformation to occur;
(b) contacting each microdroplet in turn with the target molecule at the given location and for a given period of time under conditions where the chemical transformation can occur, wherein the method further comprising the step of synchronising the microdroplets flow over the particle;
(c) at the end of the given period removing the microdroplet from the given location;
wherein the microdroplets are caused to move over a surface chemically modified to have areas of locally increased level of wettability and wherein such an increase in wettability is used to mediate the wetting of the microdroplet around the target molecule; and
wherein the electrowetting or optically-mediated electrowetting effects are used to locally modify the wettability of a region of the surface around the particle.

2. The method according to claim 1, wherein the microdroplets are suspended in an immiscible carrier fluid.

3. The method according to any one of the preceding claims, wherein the microdroplets are driven by one or a combination of pressure driven flow, electrowetting or optically-mediated EWOD.

4. The method according to any one of the preceding claims, wherein step (c) further comprising the step of creating a stream of microdroplets and transporting the microdroplets along a pathway of a microfluidic space.
